# EUROPEAN PATENT APPLICATION

(11) **EP 4 219 481 A1**
(43) Date of publication of application: **02.08.2023**
(21) Application number: 22861571.2
(22) Date of filing: 27.07.2022
(51) Int. Cl.: C07D 403/14, C07D 251/24, H01M 4/60, H01M 10/052

(54) **POROUS SOLID COMPOUND, METHOD FOR PREPARING SAME, CATHODE FOR LITHIUM SECONDARY BATTERY COMPRISING POROUS SOLID COMPOUND, AND LITHIUM SECONDARY BATTERY**

(30) Priority: 25.08.2021 KR 20210112042
(71) Applicant: LG Energy Solution, Ltd., Seoul 07335 (KR)
(72) Inventor: KIM, Yonghwi, Daejeon 34122 (KR); LEE, Changhoon, Daejeon 34122 (KR)
(74) Representative: Goddar, Heinz J.
(86) International application number: PCT/KR2022/011023
(87) International publication number: WO 2023/027359

(57) **Abstract**

Disclosed is a porous solid compound having high porosity prepared by controlling the reaction conditions of a compound containing a cyano group and a halogenated metal compound, its preparation method, a positive electrode for a lithium secondary battery comprising the porous solid compound, and a lithium secondary battery. The porous solid compound comprises one or more heterocycles formed by alternately bonding triazine and phenyl or biphenyl wherein the pore volume of the porous solid compound is 5 cm³/g or more.

## Description

### [Technical Field]

This application claims the benefit of priority based on Korean Patent Application No. 10-2021-0112042 filed on August 25, 2021, all contents of which are incorporated herein by reference.

The present invention relates to a porous solid compound, its preparation method, a positive electrode for a lithium secondary battery containing the porous solid compound, and a lithium secondary battery, and more particularly to a porous solid compound having high porosity prepared by controlling the reaction conditions of a compound containing a cyano group and a halogenated metal compound, its preparation method, a positive electrode for a lithium secondary battery containing the porous solid compound, and a lithium secondary battery.

### [Background Art]

As the interest in energy storage technology continues to increase, since its application field is expanding from energy for mobile phones, tablets, laptops and camcorders to even energy for electric vehicles (EVs) and hybrid electric vehicles (HEVs), research and development of electrochemical devices are gradually increasing. The field of electrochemical devices is an area that is receiving the most attention in this respect. Among them, the development of lithium-based secondary batteries such as a lithium-sulfur battery capable of being charged/discharged has become a focus of attention. In recent years, in developing these batteries, in order to improve capacity density and specific energy, it has led to research and development in designs for new electrodes and batteries.

This lithium-based secondary battery contains a porous carbon-based material in the positive electrode to have excellent electrical conductivity without causing side reactions in the internal environment of the battery and without causing chemical changes in the battery. That is, the carbon-based material has a porous structure or a high specific surface area, and for example, may be at least one selected from the group consisting of graphite; graphene; carbon blacks such as Denka black, acetylene black, Ketjen black, channel black, furnace black, lamp black, and thermal black; carbon nanotubes (CNTs) such as single wall carbon nanotube (SWCNT), and multiwall carbon nanotubes (MWCNT); carbon fibers such as graphite nanofiber (GNF), carbon nanofiber (CNF), and activated carbon fiber (ACF); and activated carbon, and its shape may be spherical, rod-shaped, needle-shaped, plate-shaped, tubular or bulk-shaped.

However, the carbon-based materials exemplified above do not have high porosity (i.e., a pore volume of 5 cm³/g or more, an average pore size of 25 nm or more, and a specific surface area of 1,000 m²/g or more), and thus have limitations in maximizing the performance of the lithium-based secondary battery. For example, in the case of a lithium-sulfur battery, if the porosity of the carbon material is lower than the above, the content of sulfur that can be supported is lowered and thus the energy density of the battery is lowered, and if the specific surface area is lower than the above, the reactivity of the supported sulfur is lowered, and thus output characteristics and energy density are lowered.

In this regard, although continuous research has been carried out in this industry to develop a porous carbon material with high porosity, it has yet to yield any remarkable results. Therefore, it is required to develop a new carbon material for a positive electrode that has high porosity (i.e., a pore volume of 5 cm³/g or more, an average pore size of 25 nm or more, and a specific surface area of 1,000 m²/g or more) and thus can maximize the performance of a lithium-based secondary battery.

### [Disclosure]

### [Technical Problem]

Accordingly, it is an object of the present invention to provide a porous solid compound having high porosity prepared by controlling the reaction conditions of a compound containing a cyano group and a halogenated metal compound, its preparation method, a positive electrode for a lithium secondary battery comprising the porous solid compound, and a lithium secondary battery.

### [Technical Solution]

In order to achieve the above object, the present invention provides a porous solid compound comprising one or more heterocycles formed by alternately bonding triazine and phenyl or biphenyl, and having a pore volume of 5 cm³/g or more.

In addition, the present invention provides a method for preparing a porous solid compound, comprising the step of preparing a porous solid compound by reacting a monomer containing two or more cyano groups in the presence of a halogenated metal compound catalyst, wherein the reaction is carried out at a temperature of exceeding 700 °C to 800 °C or less.

In addition, the present invention provides a positive electrode for a lithium secondary battery comprising the porous carbon material containing the porous solid compound as a positive electrode active material.

In addition, the present invention provides a lithium secondary battery comprising the positive electrode for the lithium secondary battery; a lithium metal negative electrode; an electrolyte interposed between the positive electrode and the negative electrode; and a separator.

### [Advantageous Effects]

According to the porous solid compound, its preparation method, the positive electrode for the lithium secondary battery containing the porous solid compound and the lithium secondary battery according to the present invention, it is possible to prepare a porous solid compound with high porosity by reacting a compound containing a cyano group (-CN) with a halogenated metal compound at a specific temperature, time and concentration range, and it has the advantage of improving the performance of the lithium secondary battery by applying this to the positive electrode.

### [Description of Drawings]

FIG. 1a and 1b are actual images of the porous solid compound prepared according to an Example of the present invention.
FIG. 2a is an image before reaction of the reactant used in the present invention, and FIG. 2b is an actual image of a porous solid compound prepared according to another Example of the present invention.
FIG. 3 is a graph showing the porosity of porous solid compounds prepared according to an Example of the present invention and a Comparative Example.
FIG. 4 is a graph showing the pore size distribution of porous solid compounds prepared according to an Example of the present invention and a Comparative Example.
FIG. 5 is a graph showing the porosity of porous solid compounds prepared according to an Example of the present invention and Comparative Examples.
FIG. 6 is a graph showing the pore size distribution of porous solid compounds prepared according to an Example of the present invention and Comparative Examples.
FIG. 7 is a graph showing the porosity of the porous solid compound prepared according to Comparative Examples.
FIG. 8 is a graph showing the pore size distribution of the porous solid compound prepared according to Comparative Examples.

### [Best Mode]

Hereinafter, the present invention will be described in detail.

The porous solid compound according to the present invention comprises one or more heterocycles formed by alternately bonding triazine and phenyl or biphenyl, and is characterized in that the pore volume is 5 cm³/g or more.

Until now, the lithium-based secondary battery uses a conventional carbon material such as graphite; graphene; carbon blacks such as Denka black, acetylene black, Ketjen black, channel black, furnace black, lamp black, and thermal black; carbon nanotubes (CNTs) such as single wall carbon nanotube (SWCNT), and multiwall carbon nanotubes (MWCNT); carbon fibers such as graphite nanofiber (GNF), carbon nanofiber (CNF), and activated carbon fiber (ACF); and activated carbon as a carbon-based material included in the positive electrode, but none of these carbon materials have high porosity (i.e., a pore volume of 5 cm³/g or more, an average pore size of 25 nm or more, and a specific surface area of 1,000 m²/g or more), and thus there is a limit to maximizing the performance of the lithium-based secondary battery.

In this regard, although continuous research has been carried out in this industry to develop a porous carbon material with high porosity, it has yet to yield any remarkable results. However, the applicant of the present invention has prepared a porous solid compound having high porosity (i.e., a pore volume of 5 cm³/g or more, an average pore size of 25 nm or more, and a specific surface area of 1,000 m²/g or more) by reacting a compound containing a cyano group (-CN) with a halogenated metal compound at a specific temperature, time, and concentration range.

Specifically, the porous solid compound of the present invention includes one or more heterocycles formed by alternately bonding triazine and phenyl or biphenyl, wherein the hexagonal heterocycle of Formula 1 below formed by alternately bonding triazine and phenyl and the hexagonal heterocycle of Formula 2 below formed by alternately bonding triazine and biphenyl may be exemplified as the heterocycle, but the present invention is not limited thereto.

More specifically, the porous solid compound of the present invention is characterized in that it has the structure of Formula 1-1 below, in which heterocycles of the same type starting with the heterocycle represented by Formula 1 below are continuously formed in honeycomb form, or has the structure of Formula 2-1 below, in which heterocycles of the same type starting with the heterocycle represented by Formula 2 below are continuously formed in honeycomb form (A continuous structure is formed in honeycomb form through the dotted line portions indicated in each of Formulas 1 and 2 below).

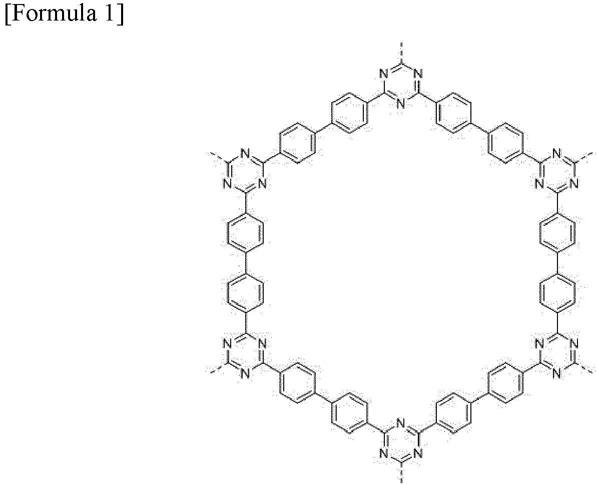

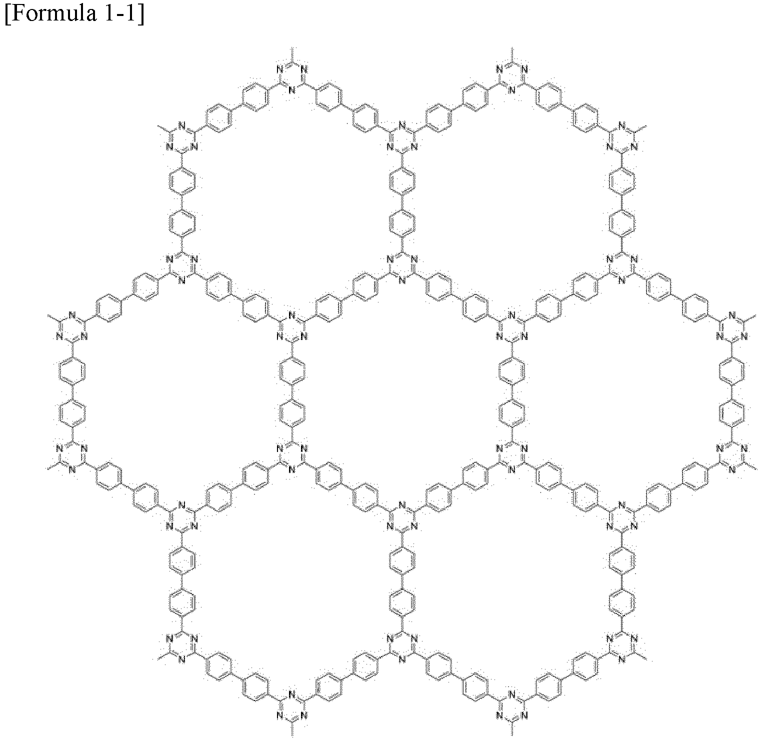

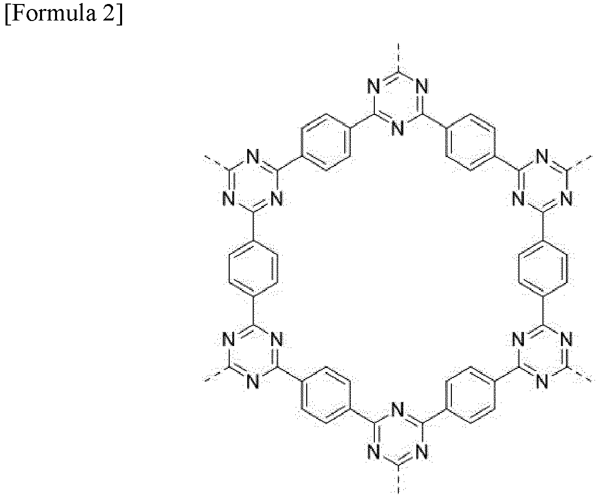

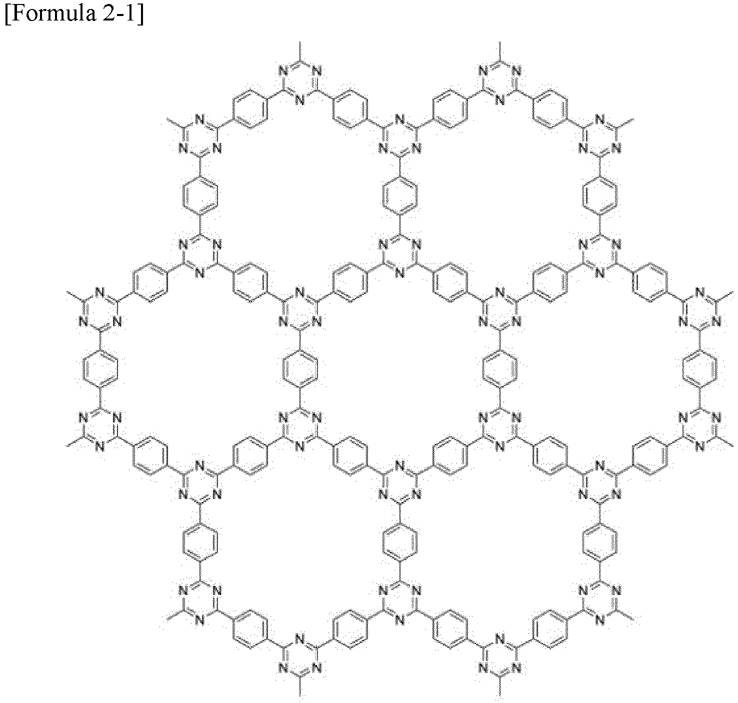

Meanwhile, when phenyl or biphenyl, which is a reactant (i.e., which is described in detail in the monomer containing two or more cyano groups and the preparation method of the porous solid compound to be described later) used to prepare the heterocycle of Formula 1 and Formula 2, is substituted with a hetero atom, any one or more of phenyl comprised in the heterocycle of Formulas 1 and 2 may be substituted with a hetero atom. In addition, at least one of the triazines comprised in the heterocycles of Formulas 1 and 2 may be substituted with a linear, branched and/or cyclic saturated or unsaturated hydrocarbon group having 1 to 20 carbon atoms comprising or not comprising 1 to 20 hetero atoms.

Meanwhile, as described above, the porous solid compound of the present invention may have a pore volume of 5 cm³/g or more, preferably 5 to 10 cm³/g, and more preferably 5 to 7 cm³/g. If the pore volume of the porous solid compound is less than 5 cm³/g, the overall porosity may be lowered, and thus the performance of the lithium secondary battery to which the porous solid compound is applied as a positive electrode active material may be deteriorated. For example, if a porous solid compound having a pore volume of less than 5 cm³/g is applied as a positive electrode active material of a lithium-sulfur battery, the content of supported sulfur may be lowered, and thus the energy density of the battery may be lowered. In addition, since the remaining space after the loading of sulfur is small, the reactivity of the positive electrode is reduced, which may lead to a problem in which output characteristics and energy density are lowered.

In addition, the porous solid compound of the present invention may have an average pore size (i.e., average diameter of pores) of 25 nm or more, preferably 30 to 70 nm, and more preferably 40 to 55 nm. If the pore size of the porous solid compound is less than 25 nm, the overall porosity may be lowered, and thus the performance of a lithium secondary battery to which the porous solid compound is applied as a positive electrode active material may be deteriorated. For example, if a porous solid compound having an average pore size of less than 25 nm is applied as a positive electrode active material of a lithium-sulfur battery, since the remaining space after the loading of sulfur is small, the reactivity of the positive electrode is lowered, and accordingly, output characteristics and energy density may be lowered.

In addition, the porous solid compound of the present invention may have a specific surface area of 1,000 m²/g or more, preferably 1,000 to 2,500 m²/g, and more preferably 1,300 to 2,200 m²/g. If the specific surface area of the porous solid compound is less than 1,000 m²/g, since the reaction area is small, the output characteristics and energy density of the lithium secondary battery to which the porous solid compound is applied as a positive electrode active material may be reduced.

That is, the carbon material comprised in the positive electrode active material of the lithium secondary battery can maximize battery performance only when it satisfies all of the pore volume (5 cm³/g or more), the average pore size (25 nm or more), and the specific surface area (1,000 m²/g or more), and the porous solid compound of the present invention satisfies all of these conditions, and thus the performance of the battery can be improved compared to a conventional carbon material.

Next, a method for preparing the above porous solid compound will be described.

The method for preparing a porous solid compound according to the present invention includes preparing a porous solid compound by reacting a monomer containing two or more cyano groups in the presence of a halogenated metal compound catalyst, wherein the reaction is carried out at a temperature of exceeding 700 °C to 800 °C or less.

The monomer containing two or more cyano groups may be a substituted or unsubstituted aromatic ring having 6 to 30 carbon atoms, which contains two or more cyano groups, or a substituted or unsubstituted aromatic heterocycle having 4 to 30 carbon atoms, which contains two or more cyano groups, and preferably is exemplified by dicyanobiphenyl 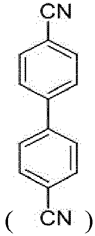 and dicyanobenzene 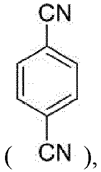 but is not limited thereto.

In addition, the halogenated metal compound (catalyst) may be, for example, any compound known as a conventional halogenated metal compound, such as zinc chloride (ZnCl₂), aluminum chloride (AlCl₃), iron chloride (FeCl₂, FeCl₃), sodium chloride (NaCl) and magnesium chloride (MgCl₂).

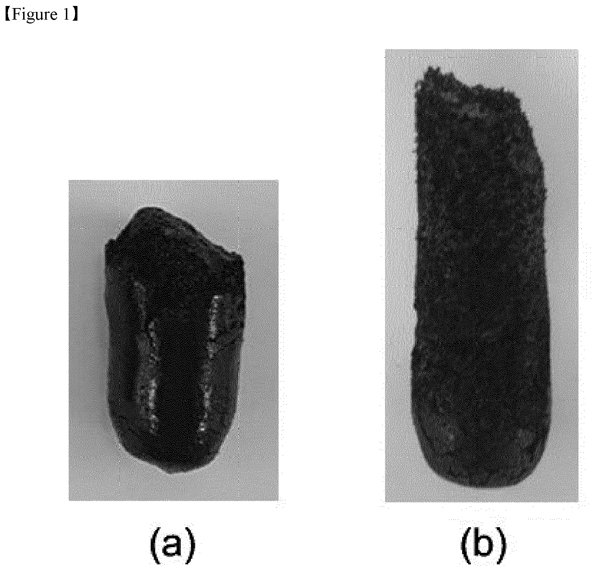

As an example, as shown in Reaction Scheme 1 below, if dicyanobiphenyl is used as the monomer containing two or more cyano groups and zinc chloride is used as the halogenated metal compound, the heterocycle compound represented by Formula 1 is prepared through a nitrile trimerization reaction. Meanwhile, FIG. 1a and 1b are actual images of the porous solid compound prepared through Reaction Scheme 1 below.

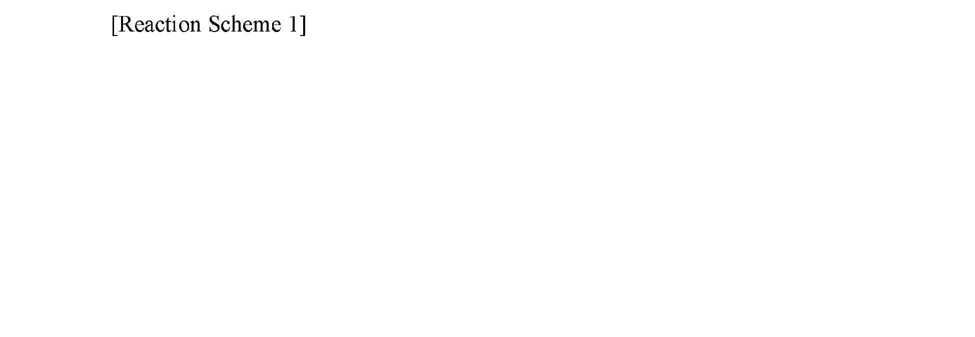

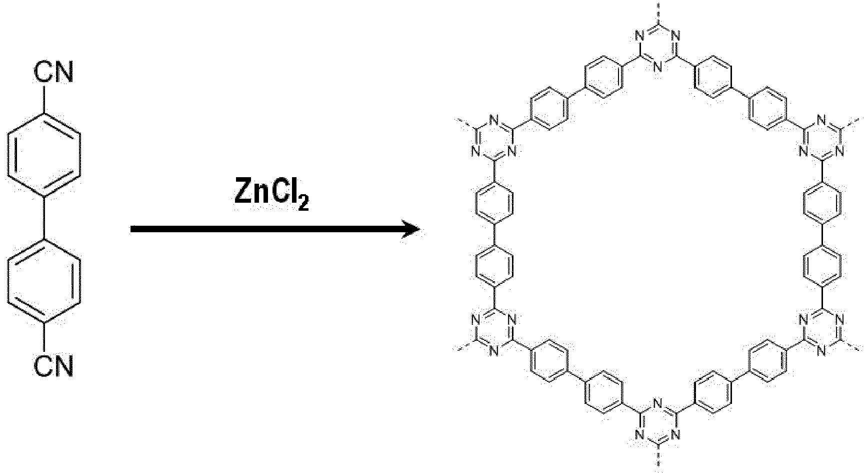

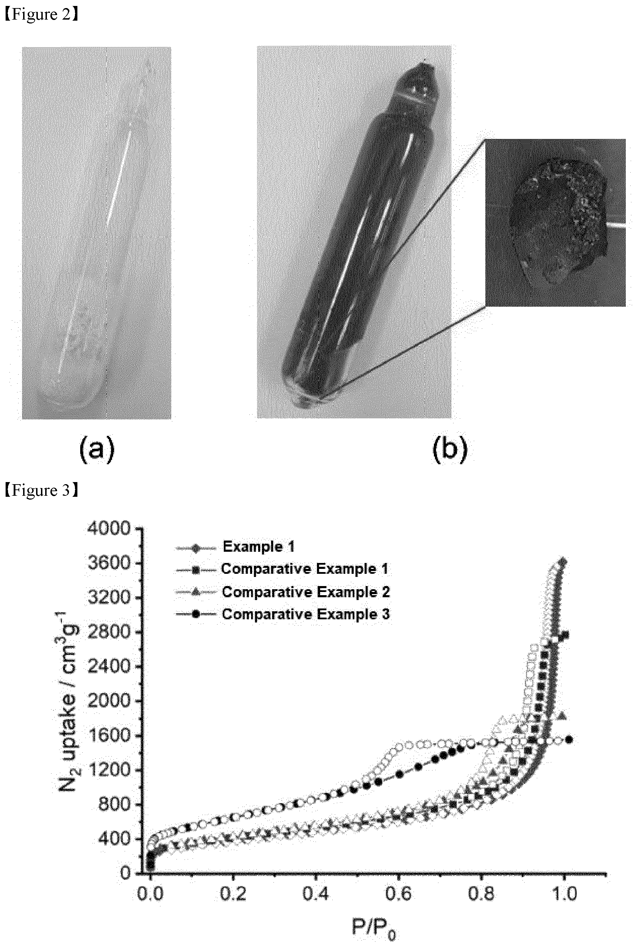

As another example, as shown in Reaction Scheme 2 below, if dicyanobenzene is used as the monomer containing two or more cyano groups and zinc chloride is used as the halogenated metal compound, the heterocycle compound represented by Formula 2 is prepared through a nitrile trimerization reaction. Meanwhile, FIG. 2a is an image before reaction of dicyanobenzene used in Reaction Scheme 2 below, and FIG. 2b is an actual image of a porous solid compound prepared through Reaction Scheme 2 below.

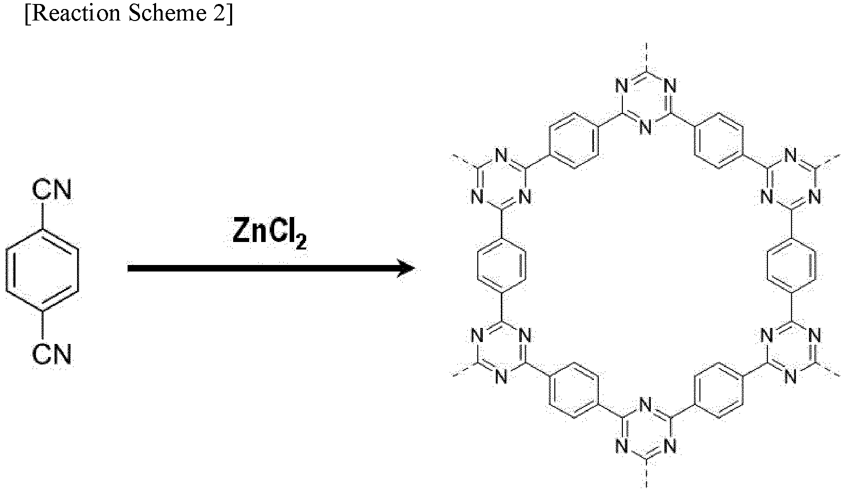

Meanwhile, in the present invention, a porous solid compound having high porosity (i.e., a pore volume of 5 cm³/g or more, an average pore size of 25 nm or more, and a specific surface area of 1,000 m²/g or more) is prepared by reacting a monomer containing two or more cyano groups in the presence of a halogenated metal compound catalyst under a specific concentration range, a specific temperature, and a specific time, and hereinafter, the concentration range and the reaction condition of the reactants will be described in detail.

The concentration of monomer containing the two or more cyano groups is 4 to 15 mmol/L, preferably 4.5 to 10 mmol/L. In particular, if the monomer containing the two or more cyano groups is dicyanobenzene, it is even more preferable that the concentration of this is 9 to 10 mmol/L.

The amount of the halogenated metal compound used relative to the monomer containing two or more cyano groups may be 5 to 25 equivalents. For example, if the monomer containing the two or more cyano groups is dicyanobiphenyl, the amount of the halogenated metal compound used relative to dicyanobiphenyl may be 18 to 25 equivalents, preferably 20 to 23 equivalents. In addition, if the monomer containing the two or more cyano groups is dicyanobenzene, the amount of halogenated metal compound used relative to dicyanobenzene may be 5 to 25 equivalents, preferably 5 to 10 equivalents.

Next, the reaction of the monomer containing two or more cyano groups with the halogenated metal compound should be carried out within a temperature range of exceeding 700 °C to 800 °C or less, preferably exceeding 700 °C to 750 °C or less. If the reaction of the monomer containing two or more cyano groups with the halogenated metal compound is performed at a temperature of 700 °C or less, the pore volume may not be sufficiently developed. In addition, if the reaction is performed at a temperature exceeding 800 °C, the reaction vessel may be damaged due to high pressure.

In addition, the reaction of the monomer containing two or more cyano groups with the halogenated metal compound should be performed within the above temperature range for 48 to 72 hours, preferably 60 to 72 hours. If the reaction of the monomer containing two or more cyano groups with the halogenated metal compound is performed in less than 48 hours, there may be a problem that the pore volume and surface area are not sufficiently formed. In addition, if the reaction exceeds 72 hours, there may be a problem that the pore volume and the surface area are not uniformly formed.

Meanwhile, after preparing the porous solid compound by reacting the monomer containing two or more cyano groups with the halogenated metal compound, the method may further comprise removing the unreacted residual halogenated metal compound with an aqueous acid solution. If the unreacted residual halogenated metal compound is not removed as described above, since there is a possibility that the specific surface area of the porous solid compound may be reduced or the purity may be lowered, it may be preferable to go through the process of removing unreacted residual halogenated metal compounds as much as possible, after preparing the porous solid compound. Meanwhile, the acid component of the aqueous acid solution may be selected from the group consisting of hydrochloric acid, nitric acid, and mixtures thereof, but is not limited thereto.

Subsequently, a positive electrode for a lithium secondary battery comprising the porous carbon material which comprises the porous solid compound as a positive electrode active material will be described. The positive electrode for the lithium secondary battery comprises the porous carbon material, which comprises the porous solid compound described above as a positive electrode active material, and may also comprise a sulfur-porous solid compound composite, in which sulfur is supported in pores of the porous solid compound, as a positive electrode active material. For the porous solid compound, the above description applies mutatis mutandis. The sulfur may be at least one selected from the group consisting of elemental sulfur (S₈), Li₂Sₙ (n≥1), an organic sulfur compound and a carbon-sulfur polymer[(C₂Sₓ)ₙ, x=2.5∼50, n≥2], and among them, it may be preferable to apply inorganic sulfur (S₈), but is not limited thereto. Meanwhile, the content of the porous solid compound included in the positive electrode for the lithium secondary battery may be 70 to 98 % by weight, preferably 75 to 90 % by weight, based on the total weight of the positive electrode for the lithium secondary battery of the present invention. In addition, the porous carbon material may comprise any one or more of porous carbon materials known in the art, for example, graphene, graphite, and carbon nanotube, in addition to the porous solid compound.

In addition, the positive electrode for the lithium secondary battery comprises a binder, and it is a component that assists in the bonding between the positive electrode active material and the electrically conductive material (only if necessary), and the binding to the current collector, and for example, may be, but is not limited to, at least one selected from the group consisting of polyvinylidenefluoride (PVdF), polyvinylidenefluoride-polyhexafluoropropylene copolymer (PVdF/HFP), polyvinylacetate, polyvinylalcohol, polyvinylether, polyethylene, polyethyleneoxide, alkylated polyethyleneoxide, polypropylene, polymethyl(meth)acrylate, polyethyl(meth)acrylate, polytetrafluoroethylene (PTFE), polyvinylchloride, polyacrylonitrile, polyvinylpyridine, polyvinylpyrrolidone, styrenebutadiene rubber, acrylonitrile-butadiene rubber, ethylene-propylene-diene monomer (EPDM) rubber, sulfonated EPDM rubber, styrene-butylene rubber, fluorine rubber, carboxymethylcellulose (CMC), starch, hydroxypropyl cellulose, regenerated cellulose, and mixtures thereof.

The binder is added in an amount of 1 to 50 parts by weight, preferably 3 to 15 parts by weight, based on 100 parts by weight of the total weight of the positive electrode. If the content of the binder is less than 1 part by weight, the adhesive strength between the positive electrode active material and the current collector may be insufficient. If the content of the binder exceeds 50 parts by weight, the adhesive strength is improved but the content of the positive electrode active material may be reduced accordingly, thereby lowering the capacity of the battery.

Meanwhile, the positive electrode for the lithium secondary battery of the present invention may further comprise an electrically conductive material if necessary. The electrically conductive material is not particularly limited as long as it does not cause side reactions in the internal environment of the lithium-sulfur battery and has excellent electrical conductivity while not causing chemical changes in the battery. The electrically conductive material may typically be graphite or electrically conductive carbon, and for example, but is not limited to, graphite such as natural graphite or artificial graphite; carbon black such as carbon black, acetylene black, Ketjen black, Denka black, thermal black, channel black, furnace black, lamp black, etc.; carbon-based materials whose crystal structure is graphene or graphite; electrically conductive fibers such as carbon fibers and metal fibers; carbon fluoride; metal powders such as aluminum powder and nickel powder; electrically conductive whiskers such as zinc oxide and potassium titanate; electrically conductive oxides such as titanium oxide; and electrically conductive polymers such as polyphenylene derivatives may be used alone or in combination of two or more thereof.

The electrically conductive material is typically added in an amount of 0.5 to 50 parts by weight, preferably 1 to 30 parts by weight based on 100 parts by weight of total weight of the positive electrode comprising the positive electrode active material. If the content of electrically conductive material is too low, that is, if it is less than 0.5 parts by weight, it is difficult to obtain an effect on the improvement of the electrical conductivity, or the electrochemical characteristics of the battery may be deteriorated. If the content of the electrically conductive material exceeds 50 parts by weight, that is, if it is too much, the amount of positive electrode active material is relatively small and thus capacity and energy density may be lowered. The method of incorporating the electrically conductive material into the positive electrode is not particularly limited, and conventional methods known in the related art such as coating on the positive electrode active material can be used. Also, if necessary, the addition of the second coating layer with electrical conductivity to the positive electrode active material may replace the addition of the electrically conductive material as described above.

In addition, a filler may be selectively added to the positive electrode of the present invention as a component for inhibiting the expansion of the positive electrode. Such a filler is not particularly limited as long as it can inhibit the expansion of the electrode without causing chemical changes in the battery, and for example, olefinic polymers such as polyethylene and polypropylene; fibrous materials such as glass fibers and carbon fibers may be used as a filler.

In addition, the positive electrode current collector may be, but is not necessarily limited to, platinum (Pt), gold (Au), palladium (Pd), iridium (Ir), silver (Ag), ruthenium (Ru), nickel (Ni), stainless steel (STS), aluminum (Al), molybdenum (Mo), chromium (Cr), carbon (C), titanium (Ti), tungsten (W), ITO (In doped SnO₂), FTO (F doped SnO₂), or an alloy thereof, or aluminum (Al) or stainless steel whose surface is treated with carbon (C), nickel (Ni), titanium (Ti) or silver (Ag) or so on. The shape of the positive electrode current collector may be in the form of a foil, film, sheet, punched form, porous body, foam or the like.

Finally, the present invention provides a lithium secondary battery comprising the positive electrode for the lithium secondary battery, a lithium metal negative electrode, an electrolyte interposed between the positive electrode and the negative electrode, and a separator. In general, a lithium secondary battery consists of a positive electrode composed of a positive electrode material and a current collector, a negative electrode composed of a negative electrode material and a current collector, and a separator that blocks electrical contact between the positive electrode and the negative electrode and allows the movement of lithium ions, and contains an electrolyte solution impregnated therein and conducting lithium ions. The negative electrode may be prepared according to a conventional method known in the art. For example, the negative electrode active material, the electrically conductive material, the binder, and, if necessary, a filler, etc. can be dispersed and mixed in a dispersion medium (solvent) to make a slurry, and the slurry can be applied onto the negative electrode current collector, followed by drying and rolling it to prepare a negative electrode.

The negative electrode active material may be a lithium metal or a lithium alloy (for example, an alloy of lithium and a metal such as aluminum, zinc, bismuth, cadmium, antimony, silicon, lead, tin, gallium or indium). The negative electrode current collector may be, but is not limited to, platinum (Pt), gold (Au), palladium (Pd), iridium (Ir), silver (Ag), ruthenium (Ru), nickel (Ni), stainless steel (STS), copper (Cu), molybdenum (Mo), chromium (Cr), carbon (C), titanium (Ti), tungsten (W), ITO (In doped SnO₂), FTO (F doped SnO₂), an alloy thereof, and copper (Cu) or stainless steel whose surface was treated with carbon (C), nickel (Ni), titanium (Ti) or silver (Ag) or so on. The shape of the negative electrode current collector may be in the form of a foil, film, sheet, punched form, porous body, foam or the like.

The separator is interposed between the positive electrode and the negative electrode and prevents a short circuit therebetween and serves as a pathway for lithium ions to move. Olefin-based polymers such as polyethylene and polypropylene, glass fibers or the like may be used in the form of sheets, multilayers, microporous films, woven fabrics, nonwoven fabrics or the like as the separator, but is not necessarily limited thereto. Meanwhile, if a solid electrolyte (e.g., an organic solid electrolyte, an inorganic solid electrolyte, etc.) such as a polymer is used as the electrolyte, the solid electrolyte may also serve as a separator. Specifically, an insulating thin film with high ion permeability and mechanical strength is used. The pore diameter of the separator is generally in the range of 0.01 to 10 *µ*m, and the thickness may generally be in the range of 5 to 300 *µ*m.

As the electrolyte solution which is a non-aqueous electrolyte solution (non-aqueous organic solvent), carbonate, ester, ether, or ketone may be used alone or in combination of two or more thereof, but is not limited thereto. For example, an aprotic organic solvent, such as dimethyl carbonate, diethyl carbonate, dipropyl carbonate, methyl propyl carbonate, ethyl propyl carbonate, methyl ethyl carbonate, ethylene carbonate, propylene carbonate, butylene carbonate, γ-butyrolactone, n-methyl acetate, n-ethyl acetate, n-propyl acetate, phosphoric acid triester, dibutyl ether, N-methyl-2-pyrrolidinone, 1,2-dimethoxyethane, tetrahydrofuran derivatives such as 2-methyltetrahydrofuran, dimethyl sulfoxide, formamide, dimethylformamide, dioxolane and derivatives thereof, acetonitrile, nitromethane, methyl formate, methyl acetate, trimethoxymethane, sulfolane, methyl sulfolane, 1,3-dimethyl-2-imidazolidinone, methyl propionate, ethyl propionate and the like can be used, but is not limited thereto.

Lithium salts may be added to the electrolyte solution (so-called non-aqueous electrolyte solution containing lithium salt). The lithium salts may comprise, but not limited to, those known to be favorably soluble in non-aqueous electrolyte solutions, for example, LiCl, LiBr, LiI, LiClO₄, LiBF₄, LiB₁₀Cl₁₀, LiPF₆, LiCF₃SO₃, LiCF₃CO₂, LiAsF₆, LiSbF₆, LiPF₃(CF₂CF₃)₃, LiAlCl₄, CH₃SO₃Li, CF₃SO₃Li, (CF₃SO₂)₂NLi, lithium chloroborane, lithium lower aliphatic carboxylate, lithium tetraphenyl borate, or lithium imide, etc. The (non-aqueous) electrolyte solution may further comprise pyridine, triethylphosphite, triethanolamine, cyclic ether, ethylene diamine, n-glyme, hexamethylphosphoric triamide, nitrobenzene derivatives, sulfur, quinoneimine dyes, N-substituted oxazolidinone, N,N-substituted imidazolidine, ethylene glycol dialkyl ether, ammonium salt, pyrrole, 2-methoxy ethanol, aluminum trichloride or the like, for the purpose of improving charging-discharging characteristics, flame retardancy, and the like. If necessary, halogen-containing solvents such as carbon tetrachloride and ethylene trifluoride may be further added to impart nonflammability, and carbon dioxide gas may be further added to improve the high-temperature conservation characteristics.

The lithium secondary battery of the present invention can be manufactured by a conventional method in the art. For example, the lithium secondary battery can be manufactured by inserting the porous separator between the positive electrode and the negative electrode, and introducing the non-aqueous electrolyte solution. The lithium secondary battery according to the present invention not only exhibits improved capacity characteristics (prevention of rapid capacity degradation) under high-speed charge/discharge cycle conditions, but also has excellent cycle characteristics, rate characteristics, and lifetime characteristics, and thus can be applied to a battery cell used as a power source for a small device, and can be particularly used suitably as a unit cell for a battery module, which is a power source for a medium and large-sized device. In this respect, the present invention also provides a battery module in which at least two lithium secondary batteries are electrically connected (in series or in parallel). It is needless to say that the number of lithium secondary batteries comprised in the battery module may be variously adjusted in consideration of the use and capacity of the battery module.

In addition, the present invention provides a battery pack in which the battery modules are electrically connected according to a conventional technique in the art. The battery module and the battery pack may be used as a power source for at least one medium and large-sized device selected from power tools; electric cars comprising an electric vehicle (EV), a hybrid electric vehicle (HEV), and a plug-in hybrid electric vehicle (PHEV); electric trucks; electric commercial vehicles; or power storage systems, but are not limited thereto.

Hereinafter, preferred examples are presented in order to facilitate understanding of the present invention. However, it will be apparent to those skilled in the art that these examples are merely illustrative of the invention, various changes and modifications can be made within the scope and spirit of the invention, and such changes and modifications are intended to fall within the scope of the appended claims.

### [Example 1] Preparation of porous solid compound (I)

First, according to Reaction Scheme 1 below, 1 g (4.9 mmol) of dicyanobiphenyl monomer (Tokyo Chemical Industry company) and 13.36 g (20 equivalents relative to the dicyanobiphenyl monomer) of zinc chloride (Sigma Aldrich company) were reacted at a temperature of 710 °C for 48 hours, and then, unreacted residual zinc chloride was removed with an aqueous nitric acid solution to prepare a porous solid compound.

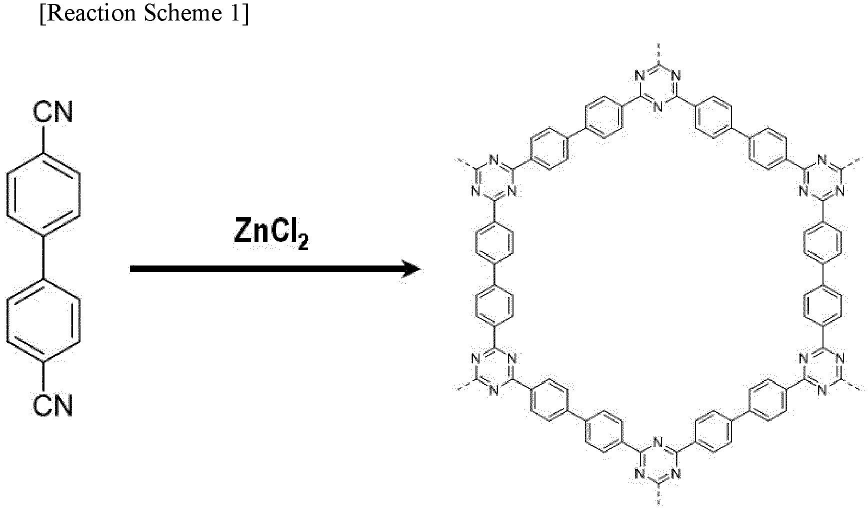

### [Comparative Examples 1 to 3] Preparation of porous solid compound (I)

The porous solid compounds of Comparative Examples 1 to 3 were prepared in the same manner as in Example 1 above, except that as shown in Table 1 below, the used amount or reaction temperature of zinc chloride (halogenated metal compound) was changed.

**Table 1:**

| | Dicyanobiphenyl (DCBP) | Zinc chloride | Reaction temperature(°C) | Reaction time(h) |
|---|---|---|---|---|
| Example 1 | 1g (4.9mmol/L) | 13.36g (20 equiv) | 710 | 48 |
| Comparative Example 1 | 1g (4.9mmol/L) | 10.02g (15 equiv) | 710 | 48 |
| Comparative Example 2 | 1g (4.9mmol/L) | 6.68g (10 equiv) | 710 | 48 |
| Comparative Example 3 | 1g (4.9mmol/L) | 13.36g (20 equiv) | 400 | 48 |

### [Experimental Example 1] Evaluation of porosity of porous solid compound (I)

In order to evaluate the porosity of the porous solid compounds prepared in Example 1 and Comparative Examples 1 to 3, the volume of the pores and the average diameter (pore size) of the pores were measured, respectively, and the specific surface area of the porous solid compound was additionally measured, and these results are shown in Table 2 below.

**Table 2:**

| | Pore volume(cm³/g) | Pore size(nm) | Specific surface area(m²/g) |
|---|---|---|---|
| Example 1 | 5.50 | 44(10∼70) | 1,380 |
| Comparative Example 1 | 4.24 | 24(15∼30) | 1,500 |
| Comparative Example 2 | 2.82 | 12(5∼15) | 1,550 |
| Comparative Example 3 | 2.14 | 3(2∼5) | 820 |

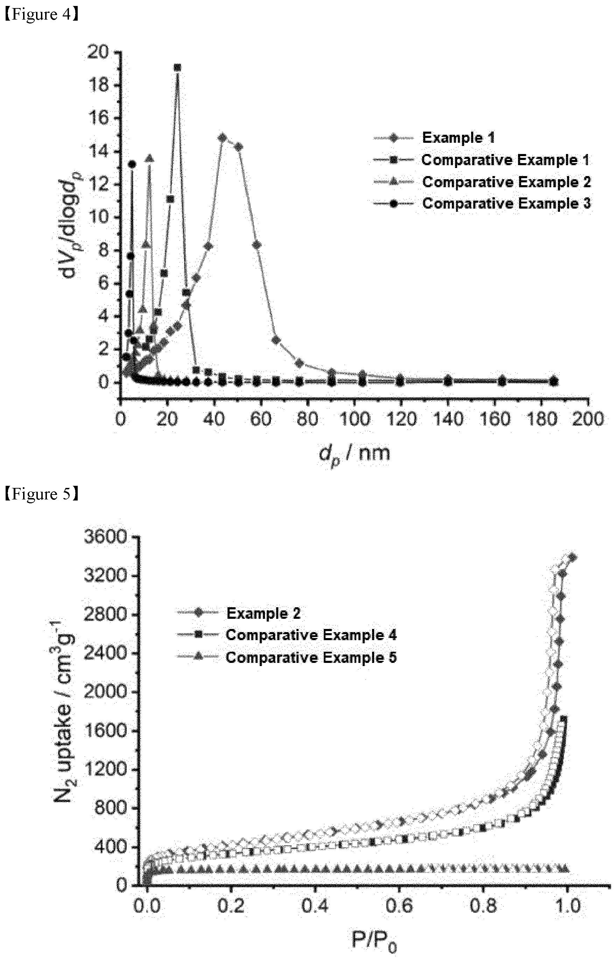

As described above, as a result of measuring the pore volume, average diameter (pore size) and specific surface area of the porous solid compounds prepared in Example 1 and Comparative Examples 1 to 3, respectively, it was confirmed that since only the porous solid compound prepared in Example 1 has a pore volume of 5 cm³/g or more, a pore size of 25 nm or more, and a specific surface area of 1,000 m²/g or more, all of the conditions aimed at the present invention are satisfied. Through this, it was confirmed that if dicyanobiphenyl is used as a reactant, the concentration, reaction temperature and reaction time of the reactant, as well as the amount of halogenated metal compound used, affect the porosity of the porous solid compound (that is, the higher the equivalent weight of the halogenated metal compound, the higher the porosity). In addition, it was found that even if the amount of halogenated metal compound used is the same, when the reaction temperature is outside the scope of the present invention (exceeding 700 °C and 800 °C or less), the porosity is lowered (corresponding to Comparative Example 3). Meanwhile, FIG. 3 is a graph showing the porosity (nitrogen adsorption and desorption isotherm) of porous solid compounds prepared according to an Example of the present invention and a Comparative Example, and FIG. 4 is a graph showing the pore size distribution of porous solid compounds prepared according to an Example of the present invention and a Comparative Example. In addition, the specific surface area of Example 1 is shown to be relatively small compared to Comparative Examples 1 and 2, but this is due to the fact that the pores in Example 1 are formed to be larger, and is because the surface area from the relatively small pores is calculated to be small.

### [Example 2] Preparation of porous solid compound (II)

First, according to Reaction Scheme 2 below, 2g (9.8 mmol) of dicyanobenzene monomer (Tokyo Chemical Industry company) and 10.64 g (5 equivalents relative to the dicyanobenzene monomer) of zinc chloride (Sigma Aldrich company) were reacted at a temperature of 710 °C for 72 hours, and then, unreacted residual zinc chloride was removed with an aqueous nitric acid solution to prepare a porous solid compound.

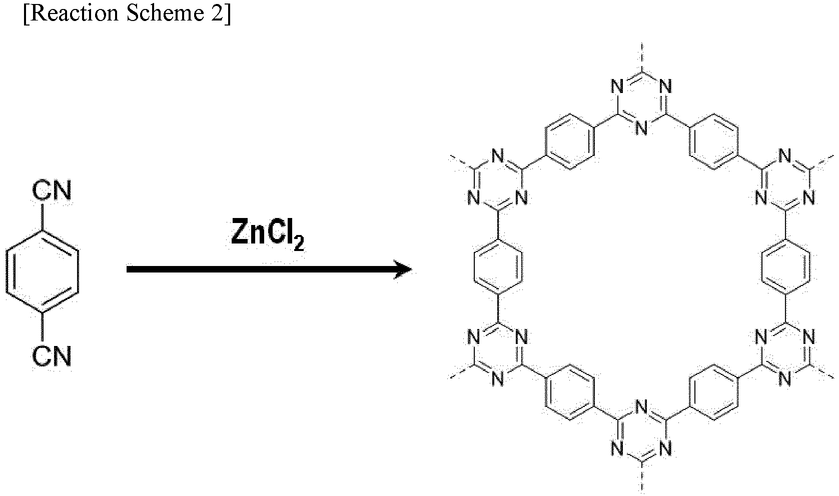

### [Comparative Examples 4 to 8] Preparation of porous solid compound (II)

The porous solid compounds of Comparative Examples 4 to 8 were prepared in the same manner as in Example 2 above, except that as shown in Table 3 below, the reaction conditions were changed.

**Table 3:**

| | Dicyanobenzene (DCB) | Zinc chloride | Reaction temperature(°C) | Reaction time(h) |
|---|---|---|---|---|
| Example 2 | 2g (9.8mmol/L) | 10.64g (5 equiv) | 710 | 72 |
| Comparative Example 4 | 2g (9.8mmol/L) | 10.64g (5 equiv) | 710 | 24 |
| Comparative | 2g | 10.64g | 400 | 72 |
| Example 5 | (9.8mmol/L) | (5 equiv) | | |
| Comparative Example 6 | 1g (4.9mmol/L) | 5.32g (5 equiv) | 710 | 72 |
| Comparative Example 7 | 1g (4.9mmol/L) | 5.32g (5 equiv) | 710 | 48 |
| Comparative Example 8 | 1g (4.9mmol/L) | 5.32g (5 equiv) | 710 | 20 |

### [Experimental Example 2] Evaluation of porosity of porous solid compound (II)

In order to evaluate the porosity of the porous solid compounds prepared in Example 2 and Comparative Examples 4 to 8, the volume of the pores and the average diameter (pore size) of the pores were measured, respectively, and the specific surface area of the porous solid compound was additionally measured, and these results are shown in Table 4 below.

**Table 4:**

| | Pore volume(cm³/g) | Pore size(nm) | Specific surface area(m²/g) |
|---|---|---|---|
| Example 2 | 5.13 | 50(15∼70) | 1,510 |
| Comparative Example 4 | 2.92 | 80(2∼150) | 1,450 |
| Comparative Example 5 | 0.46 | 1(0∼2) | 940 |
| Comparative Example 6 | 2.92 | 8.2(4∼11) | 1,950 |
| Comparative Example 7 | 2.31 | 5.5(2∼7) | 2,080 |
| Comparative Example 8 | 1.60 | 3.7(2∼5) | 1,920 |

As described above, as a result of measuring the pore volume, average diameter (pore size) of the pores and specific surface area of the porous solid compounds prepared in Example 2 and Comparative Examples 4 to 8, respectively, it was confirmed that since only the porous solid compound prepared in Example 2 has a pore volume of 5 cm³/g or more, a pore size of 25 nm or more, and a specific surface area of 1,000 m²/g or more, all of the conditions aimed at the present invention are satisfied. In particular, through Example 2 and Comparative Example 6, it was confirmed that if dicyanobenzene is used as a reactant, the porosity is lowered, even if the equivalent weight of the halogenated metal compound is the same, when the concentration of dicyanobenzene is out of the scope of the present invention. In addition, it was found that the reaction time also affects the porosity. In addition, through Example 2 and Comparative Example 5, it was found that if the reaction temperature is outside the scope of the present invention (exceeding 700 °C and 800 °C or less), the porosity is lowered.

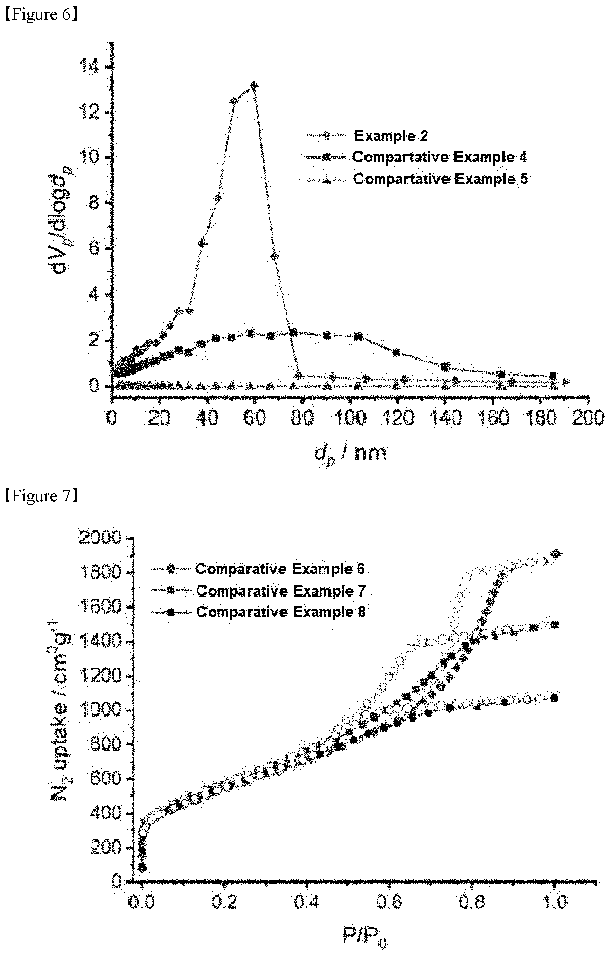

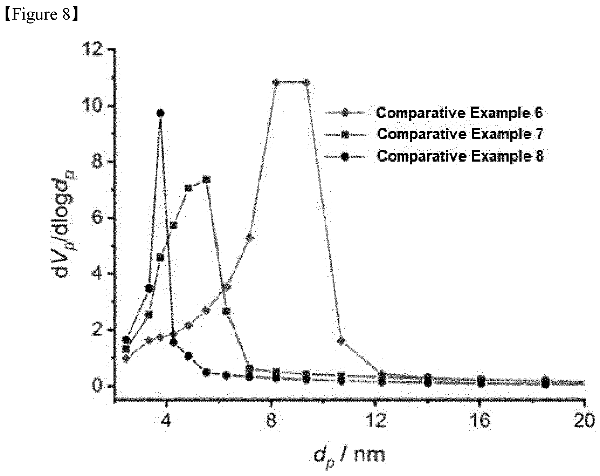

Meanwhile, FIG. 5 is a graph showing the porosity (nitrogen adsorption and desorption isotherm) of porous solid compounds prepared according to an Example of the present invention and Comparative Examples, FIG. 6 is a graph showing the pore size distribution of porous solid compounds prepared according to an Example of the present invention and Comparative Examples, and FIG. 7 is a graph showing the porosity (nitrogen adsorption and desorption isotherm) of porous solid compound prepared according to Comparative Examples, and FIG. 8 is a graph showing the pore size distribution of porous solid compounds prepared according to Comparative Examples. In addition, the specific surface area of Example 2 is shown to be relatively small compared to Comparative Examples 6 to 8, but this is due to the fact that the pores in Example 2 are formed to be larger, and is because the surface area from the relatively small pores is calculated to be small.

## Claims

1. A porous solid compound, comprising one or more heterocycles formed by alternatively bonding triazine and phenyl or biphenyl,
wherein the pore volume of the porous solid compound is 5 cm³/g or more.

2. The porous solid compound according to claim 1, wherein the porous solid compound has an average pore size of 25 nm or more.

3. The porous solid compound according to claim 1, wherein the porous solid compound has a specific surface area of 1,000 m²/g or more.

4. The porous solid compound according to claim 1, wherein the porous solid compound has the structure of Formula 1-1 below, in which heterocycles of the same type starting with the heterocycle represented by Formula 1 below are continuously formed in honeycomb form, or has the structure of Formula 2-1 below, in which heterocycles of the same type starting with the heterocycle represented by Formula 2 below are continuously formed in honeycomb form.

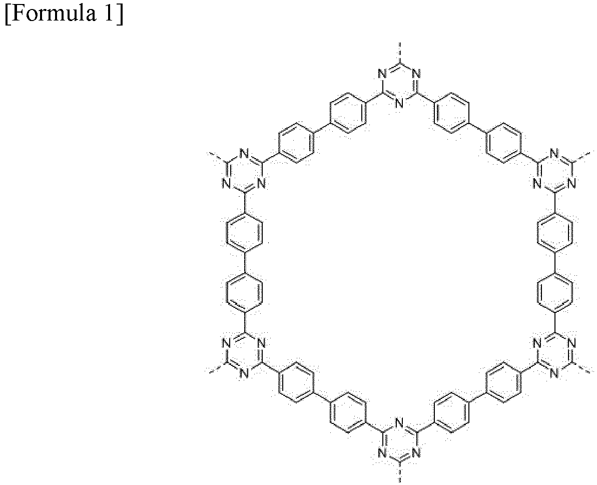

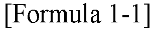

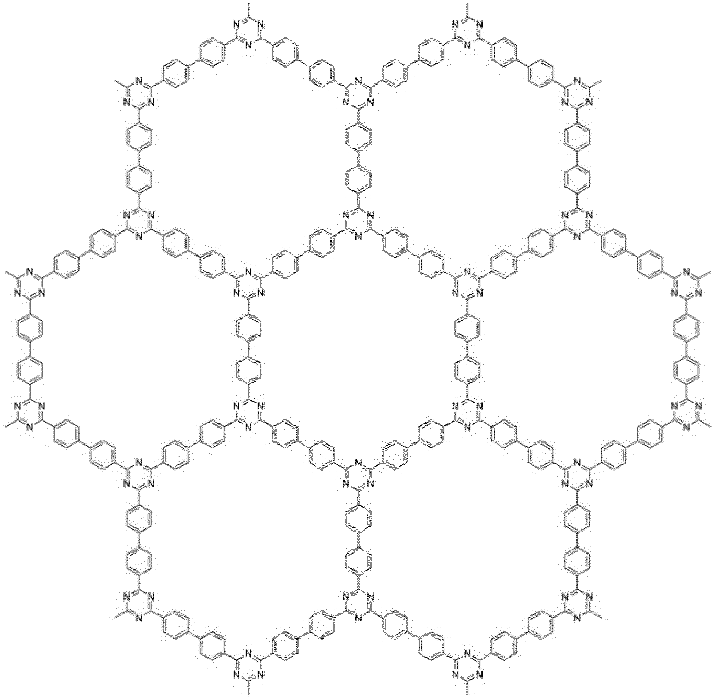

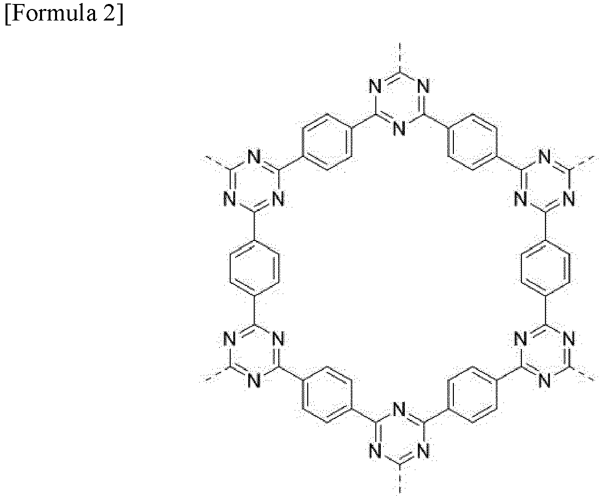

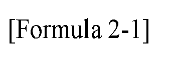

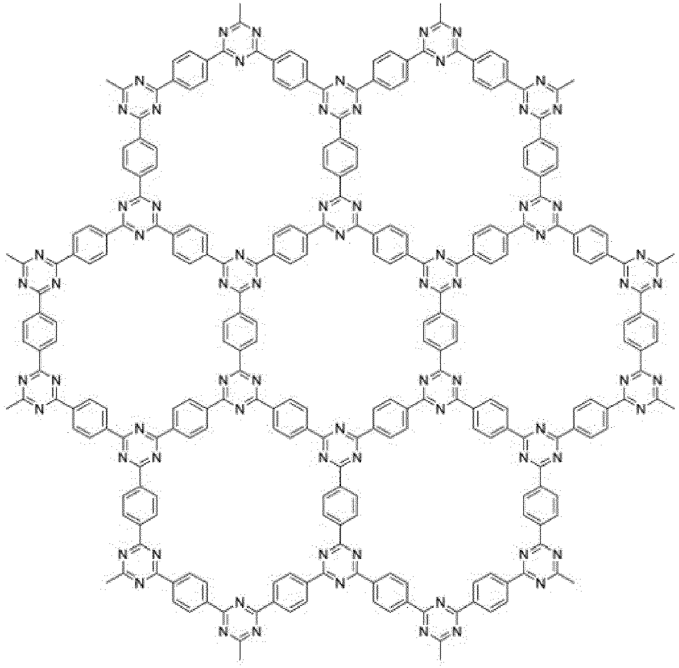

5. A method for preparing a porous solid compound comprising the step of preparing a porous solid compound by reacting a monomer containing two or more cyano groups in the presence of a halogenated metal compound catalyst,
wherein the reaction is carried out at a temperature of exceeding 700 °C to 800 °C or less.

6. The method for preparing a porous solid compound according to claim 5, wherein the monomer containing two or more cyano groups is a substituted or unsubstituted aromatic ring having 6 to 30 carbon atoms, which contains two or more cyano groups, or a substituted or unsubstituted aromatic heterocycle having 4 to 30 carbon atoms, which contains two or more cyano groups.

7. The method for preparing a porous solid compound according to claim 6, wherein the monomer containing two or more cyano groups is dicyanobiphenyl or dicyanobenzene.

8. The method for preparing a porous solid compound according to claim 5, wherein the halogenated metal compound is selected from the group consisting of zinc chloride (ZnCl₂), aluminum chloride (AlCl₃), iron chloride (FeCl₂, FeCl₃), sodium chloride (NaCl) and magnesium chloride (MgCl₂).

9. The method for preparing a porous solid compound according to claim 7, wherein the monomer containing two or more cyano groups is dicyanobiphenyl, and in this case, dicyanobiphenyl is added to the reaction at a concentration of 4 to 15 mmol/L, and the halogenated metal compound is added to the reaction at 18 to 25 equivalents relative to dicyanobiphenyl.

10. The method for preparing a porous solid compound according to claim 7, wherein the monomer containing two or more cyano groups is dicyanobenzene, and in this case, dicyanobenzene is added to the reaction at a concentration of 9 to 10 mmol/L, and the halogenated metal compound is added to the reaction at 5 to 25 equivalents relative to dicyanobenzene.

11. The method for preparing a porous solid compound according to claim 5, wherein the reaction is carried out for 48 to 72 hours.

12. The method for preparing a porous solid compound according to claim 5, further comprising a step of removing the unreacted residual halogenated metal compound with an aqueous acid solution after the step of preparing the porous solid compound.

13. A positive electrode for a lithium secondary battery comprising a porous carbon material comprising the porous solid compound of claim 1 as a positive electrode active material.

14. A lithium secondary battery comprising the positive electrode for the lithium secondary battery of claim 13; a lithium metal negative electrode; an electrolyte interposed between the positive electrode and the negative electrode; and a separator.
